# EUROPÄISCHE PATENTANMELDUNG

(11) **EP 2 263 464 A1**
(43) Veröffentlichungstag der Anmeldung: **22.12.2010**
(21) Anmeldenummer: 10165411.9
(22) Anmeldetag: 09.06.2010
(51) Int. Cl.: A01N 59/16, A47C 27/00, A61L 2/238, A61L 15/44, C08J 9/00, C08J 9/32, C08K 3/00, C08K 7/14, C08K 7/20, C08K 7/28, C08K 9/02

(54) **Verfahren zur Herstellung eines antibakteriellen PUR-Schaumstoffes**

(30) Priorität: 19.06.2009 DE 102009029948
(71) Anmelder: Eurofoam Deutschland GmbH, 66450 Bexbach/Saar (DE)
(72) Erfinder: Bettinger, Herbert, 66450, Bexbach/Saar (DE)
(74) Vertreter: Rasch, Michael

(57) **Zusammenfassung**

Die Erfindung betrifft ein Verfahren zur Herstellung eines PUR-Schaumstoffes unter Verwendung einer Suspension aus Polyol, Isocyanat und Wasser, wobei der PUR-Schaumstoffe durch Polyaddition und Bildung von Kohlendioxid gebildet wird und der Suspension silberdotierte Glaspartikel zugemengt werden. Die darin enthaltenen Ag+-Ionen stören wichtige Zellfunktion von Bakterien und Pilzen, wodurch die Zellteilung behindert wird und die Organismen absterben. Damit eignen sich derartige Schaumstoffe vor allem zur Anwendung für Matratzen im medizinischen Bereich.

## Beschreibung

Die Erfindung betrifft ein Verfahren zur Herstellung eines PUR-Schaumstoffes nach dem Oberbegriff des Anspruchs 1 sowie einen daraus hergestellten PUR Weichschaumstoff.

Polyurethan-Weichschaumstoffe (PUR) werden vielfach für Matratzen und ähnliche Unterlagen verwendet. Solche Matratzen aus PUR werden meist für Zeiträume im Bereich von 10 Jahren eingesetzt mit der Folge, dass sich mit der Zeit Bakterien und unerwünschte Keine bilden bzw. ansammeln und es schwierig bzw. aufwändig ist, diese zu beseitigen.

Denn in einer Matratze sammeln sich innerhalb von 6 Monaten bis zu 1 Millionen Milben. Dabei bilden Bakterien, Viren und Pilzsporen das Biotop in der Matratze für die Milben. Heutzutage sind Milben wiederum mit ca. 25 % der häufigste Allergieauslöser. Aber auch die Bakterien und Pilze in der Matratze beanspruchen die Gesundheit oder gefährden sie wie z.B. der MRSA. Die in der Matratze häufig vorkommenden Bedingungen wie Feuchtigkeit und Wärme sind wiederum die optimalen Wachstumsbedingungen für Pilze, Viren und Bakterien.

Die bislang angebotenen Lösungen gegen den bakteriellen Befall und Befall von Milben von Matratzen zielen auf eine Feuchte- und Klimaregulierung hin. Der Nachteil dieser Lösungsansätze besteht zweifelsohne darin, dass die abgegebene Feuchtigkeit im Schlaf letzten Endes nicht einfach absorbiert und tagsüber wieder getrocknet werden kann. Durch natürliches Schwitzen verliert jeder Mensch bis zu 1,5 Liter pro Nacht. Die im Schweiß enthaltenen Salze, Eiweiße und Schuppen dringen weiter in die Matratzen ein und verbleiben dort als Nährboden für die Bakterien, Pilze und Viren, die wiederum wichtig sind für die Nährstoffkette der Milben.

Die antibakterielle Wirkung wird bisher hauptsächlich in den Matratzen-Bezügen oder -auflagen erzielt. Der Nachteil dieser Matratzenschoner ist, dass er nicht verhindern kann dass die Feuchtigkeit des Schlafenden durch die Auflage bis in die Matratze vordringt und dort wiederum das Bakterien Wachstum beginnt. Daher gibt es im Markt eine ganze Vielzahl an Reinigungssystemen, die vor allem im gewerblichen Bereich (Hotels etc.) alle 6 bis 24 Monate angewendet werden, wobei erhebliche Reinigungskosten je Matratze entstehen.

Es ist dazu bekannt, Polyurethan-Schaumstoffen Chemikalien in flüssiger oder pulveriger Form oder Silbersalze in flüssiger Form bei dem Schäumprozess zuzudosieren und dabei diese Chemikalien in die Schaumstoffmatrix einzubauen und damit antibakterielle oder antifungizide Eigenschaften des Kunststoffes zu erzeugen. Nachteilig dabei ist, dass viele biozide Chemikalien schädliche Nebenwirkungen auf den menschlichen Organismen haben und dementsprechend Anwendungsvorschriften genau eingehalten werden müssen. Darüber hinaus verlieren die Chemikalien mit der Zeit ihre Wirksamkeit. Auch die Entsorgung derartiger chemisch belasteter Matratzen stellt ein Problem dar.

Es sind Pulver aus offenporigen oder geschlossenporigen Gläsern bzw. Glaskeramiken bekannt, bei denen Zink und/oder Silber in ionisierter Form bzw. als Nanopartikel enthalten sind. Diese Bestandteile wirken antimikobiell bzw. antibakteriell. Derartige Pulver können bei der Kunststoffherstellung beigemengt werden mit der Folge, dass das produzierte Kunststoffteil mehr oder weniger antimikobielle bzw. antibakterielle Eigenschaften aufweist. Eine Anwendung auf Schaumstoffe ist nicht bekannt.

Hiervon ausgehend liegt der Erfindung die Aufgabe zugrunde, ein Verfahren zur Herstellung von antibakteriellen oder antifungiziden PUR-Schaumstoffen bzw. einen antibakteriellen oder antifungiziden PUR-Schaumstoff bereitzustellen, welches bzw. welcher die o.g. Nachteile vermeidet. Erfindungsgemäß wird diese Aufgabe durch die in den unabhängigen Ansprüchen angegebenen Merkmale gelöst. Vorteilhafte Weiterbildungen ergeben sich aus den Unteransprüchen.

Die Herstellung von Polyurethan-Schaumstoffen erfolgt üblicherweise dadurch, dass Polyol und Isocyanat und Wasser zur Bildung einer Suspension vermischt werden. Nach wenigen Sekunden beginnt die chemische Polyadditionsreaktion, bei der Harnstoff entsteht und Kohlendioxid frei wird und dabei blähend und schaumbildend wirkt. Ferner werden üblicherweise Hilfsstoffe wie Katalysatoren (beispielsweise Amine), Emulgatoren, Farben und Schaumstabilisatoren zugegeben.

Erfindungsgemäß werden der Suspension silberdotierte Glaspartikel zugemengt, wobei diese vorzugsweise in 0,1 bis 2 Gew.-% dem Polyol beigemengt werden. Dies bewirkt eine sehr feine Verteilung der silberdotierten Glaspartikel in der Schaumstoffmatrix, was zu einer hohen Wirksamkeit gegen Bakterien und Pilze führt.

Bakterien und Pilze wachsen bei Einwirkung von Feuchtigkeit wobei ebenfalls bei Feuchtigkeit die Freisetzung von Silberionen (Ag⁺-Ionen) aus den silberdotierten Glaspartikeln erfolgt. Diese Ag⁺-Ionen stören wichtige Zellfunktion der Mikroorganismen, wodurch die Zellteilung behindert wird und die Organismen absterben.

Damit eignen sich derartige Schaumstoffe vor allem zur Anwendung für Matratzen im medizinischen Bereich, insbesondere für Krankenhausbetten. Der so erhaltende Schaumstoff entspricht vorteilhafterweise der Chemikalien-Ozonschichtverordnung (ChemOzonSchichtV).

Vorzugsweise haben die Glaspartikel im wesentlichen eine Teilchengröße von 1 bis 10 µm. Dies ermöglicht eine sehr feine Verteilung der Partikel in der Schaumstoffmatrix. Derartige silberdotierte Glaspartikel werden dadurch erhalten, dass die Glaspartikel mit Silberionen in einem speziellen Extrusionsprozess hergestellt werden, wodurch dauerhaft ionisierte Partikel erhalten werden.

Eine vorteilhafte Weiterbildung sieht vor, dass die Glaspartikel zusätzlich Zink enthalten.

Ein bevorzugtes Verfahren zur Herstellung von PUR wird nachfolgend beschrieben:
Als Polyole (Polyalkohol) werden verwendet:
   Standardpolyol, 46-50 mg KOH/g, 600-800 mPa.s oder gefülltes SAN Polyol, 10 - 45 % SAN Feststoffanteil oder reaktives HR Polyol 6000 MW, 26-30 mg KOH/g, 1000-1200 mPa.s oder reaktives HR SAN Polyol, 23-30 mg KOH/g, 1600-2600 mPa.s oder
   reaktives HR Polyol (6 funktional), 27,5 - 31,5 mg KOH/g, 1400-1600 mPa.s oder reaktives HR PHD Polyol, 26-30 mg KOH/g, 3200-4000 mPa.s oder Hypersoftpolyol, 31 - 45 mg KOH/g, 440 - 1000 mPa.s oder Polyolmischungen zur Herstellung viskoelastischer Weichschaumstoffe.

Dem Polyol werden silberdotierte Glaspartikel mit einer mittleren Teilchengröße von 5 µm in einem Verhältnis von 0,3 Gew.-% zugemengt zur Bildung einer Polyol-Glaspartikel-Suspension.

Der Polyol-Glaspartikel-Suspension werden Isocyanat und Wasser in einem Mengenverhältnis (auf 100 Teile Polyol) von Icocyanat 54 Teile und Wasser 5 Teile bei Raumgewicht Netto 19 kg/m³ bis Icocyanat 17,5 Teile und Wasser 1,05 Teile bei Raumgewicht Netto 80 kg/m³ zugegeben.

Das verwendete Isocyanat hat folgende Eigenschaften: TDI 80 , Isomerenverhältnis (2,4 Isomer) 79,5 - 81,5 und TDI 65, Isomerenverhältnis (2,4 Isomer) 64 - 68 oder MDI Präpolymer , NCO Gehalt 28 - 33, 33 - 80 mPa.s und MDI (roh), NOC Gehalt 30 - 32, 180 - 260 mPa.s

So wird z.B. in einem bevorzugten Ausführungsbeispiel in eine Formulierung aus reaktives HR Polyol 6000 MW, 26-30 mg KOH/g, 1000-1200 mPa.s plus reaktives HR SAN Polyol, 23-30 mg KOH/g, 1600-2600 mPa.s plus reaktives HR Polyol (6 funktional), 27,5 - 31,5 mg KOH/g, 1400-1600 mPa.s plus TDI 80 , Isomerenverhältnis (2,4 Isomer) 79,5 - 81,5 plus Standardpolyol, 46-50 mg KOH/g, 600-800 mPa.s mit 0,3 Gew.% zugemischten Glaspartikel mit Silberionen plus Wasser , 1,95 Teile (auf 100 Teile Polyol), dazu Katalysatoren (Amine) , Stabilisatoren und Farbstoff zu einen PUR Weischschaumstoff mit einem Raumgewicht zw. 45 und 49 kg/m³ verarbeitet.

Die Komponenten werden in einem Mischrohr miteinander vermengt, wobei nach wenigen Sekunden die Gasbildungsreaktion

R-NCO + H₂O -> R-NH₂ + CO₂

einsetzt, die je nach Raumgewicht bis zu 230 sek. dauert. Das sich abspaltende gasförmige CO₂ wirkt dabei aufblähend. Nach Abschluss wird ein PUR-Weichschaum mit einer Dichte von 19 - 80 kg/m³ erhalten, der die dotierten Glaspartikel homogen verteilt in seiner Matrix enthält.

## Patentansprüche

1. Verfahren zur Herstellung eines PUR-Schaumstoffes unter Verwendung einer Suspension aus Polyol, Isocyanat und Wasser, wobei der PUR-Schaumstoffe durch Polyaddition und Bildung von Kohlendioxid gebildet wird, **dadurch gekennzeichnet, dass** der Suspension silberdotierte Glaspartikel zugemengt werden.

2. Verfahren nach Anspruch 1, **dadurch gekennzeichnet, dass** die Glaspartikel dem Polyol beigemengt werden

3. Verfahren nach Anspruch 1, **dadurch gekennzeichnet, dass** die Glaspartikel eine Teilchengröße zwischen von 1 - 10 µm aufweisen.

4. Verfahren nach Anspruch 1, **dadurch gekennzeichnet, dass** die Glaspartikel in 0,1 bis 1, vorzugsweise 0,1 bis 0,3 Gew.-% dem Reaktionspartner Polyol zugemengt werden.

5. Verfahren nach Anspruch 1, **dadurch gekennzeichnet, dass** Glaspartikel mit Silber vorwiegend in ionisierter Form (Ag⁺) verwendet wird.

6. Verfahren nach Anspruch 1, **dadurch gekennzeichnet, dass** Glaspartikel verwendet werden, die zusätzlich Zink vorwiegend in ionisierter Form enthalten.

7. Verfahren nach Anspruch 1, **dadurch gekennzeichnet, dass** dem Polyol Hilfsstoffe, insbesondere Katalysatoren, Emulgatoren, Schaumstabilisatoren und/oder Farbstoffe zugemengt werden.

8. Schaumstoff, hergestellt nach dem Verfahren gemäß einem der vorherigen Ansprüche.

9. Schaumstoff, hergestellt nach dem Verfahren gemäß einem der vorherigen Ansprüche im Raumgewichtsbereich von 19 kg/m³ bis 80 kg/m³.
